# EUROPEAN PATENT APPLICATION

(11) **EP 0 835 857 A1**
(43) Date of publication of application: **15.04.1998**
(21) Application number: 96907807.0
(22) Date of filing: 13.03.1996
(51) Int. Cl.: C07C 19/045, C07C 17/02

(54) **METHOD OF OBTAINING 1,2-DICHLOROETHANE**

(71) Applicant: Caprolactam Joint-Stock Company, Dzerzhinsk 606000 (RU); Nizhny Novgorod State Technical University, Nizhny Novgorod 603000 (RU)
(72) Inventor: BODRIKOV, Ivan Vasilievich, Nizhny Novgorod, 603005 (RU); MUKHANOV, Anatoly Alexandrovich, Dzerzhinsk, 606029 (RU); GROSHEV, Gennady Leonidovich, Dzerzhinsk, 606000 (RU); KOLESNIKOV, Valery Yakovlevich, Dzerzhinsk, 606000 (RU); OREKHOV, Oleg Vladimirovich, Dzerzhinsk, 606031 606031 (RU); SPIRIDONOVA, Svetlana Vasilievna, Dzerzhinsk, 606033 (RU); MITCHURIN, Alexandr Andreevich, Dzerzhinsk, 606024 (RU); SEMANOV, Veniamin Konstantinovich, Dzerzhinsk, 606034 (RU); ZHIVODEROV, Alexandr Vasilievich, Dzerzhinsk, 606007 (RU)
(74) Representative: von Füner, Alexander, Dr.
(86) International application number: RU9600056
(87) International publication number: WO9733849

(57) **Abstract**

Ethylene dichloride is produced by interaction of chlorine with ethylene in liquid ethylene dichloride medium at the temperature from 20°C to the reaction mass boiling temperature in the presence of catalyst of MClnLm formula,where Cl-chlorine,M-iron (n=2,3),aluminium (n=3),tin (n=2,4),antimony (n=5) and L-trimethylamine (m=1,3),triethylamine (m=1,3),pyridine (m=1,2,3,4),1-(2-ethyl - chloride)trimethylammonium chloride (m=1),1-(2-ethyl chloride) - triethylammonium chloride (m=1),1-(2-ethyl chloride)pyridinium chloride (m=1),pyridinchlorohydrate (m=1).Chlorination is carried out in the reactor at the temperature of 20-75°C,then the product with the catalyst is fed to the ethylene chlorination reactor,operating at the reaction mass boiling temperature with the same catalyst from the top of which ethylene dichloride is withdrawn in the vapour state and the catalyst is taken out from the bottom and returned to the reactor operating at the temperature of 20-75°C. The process is carried out at the content of catalyst in the reaction mass equal to 0,01-10,0 wt.%.

## Description

### FIELD OF THE INVENTION

This invention relates to the process for production of halogen-substituted hydrocarbon.

### BACKGROUND OF THE INVENTION

It has been known that ethylene dichloride is produced by interaction of ethylene and chlorine at the temperature of 20-200°C and atmospheric pressure or at the pressure higher than the atmospheric one in the presence of 0,005-0,5% catalyst of Me(FeCl₄)n formula, where Me-NH₄,Na,K,Mg,n-valency of cation,introduced directly in the form of a compound or produced during the reaction from the compo - nents.The reaction is carried out in the solvent,preferably dichloroethane medium.The product is distilled off (West Germany Application 3245366,C 07 C 19/045,C 07 17/02,1984).

It has been also known that ethylene dichloride is produced in the presence of air and catalyst containing anhydrous iron chloride (111) and nitrogen containing compound.Ammonium chloride,trimethylammonium chloride,diaminoethane chlorohydrate,triethanolaminochlorohydrate are used as nitrogen containing compounds.The process is carried out at the temperature lower than the reaction mass boiling temperature 77°C (SU Patent 1250165,Cl.C 07C 17/02,1986).

It has been known the process for the production of dichloro - ethane (EDC) by the interaction of chlorine and ethylene in dichloroethane as solvent medium at 20-70°C in the presence of 0,002-0,3 wt.% catalyst, consisting of anhydrous iron trichloride and ammonia. Ready-made raw ethylene dichloride with the dissolved and suspended catalyst in it is taken from the reactor by overflow and is fed to the distillation column for separation of final product.The distillation of ethylene dichloride is carried out before the extraction of catalyst.Dichloroethane left in the bottom of the column (50-80% from the provided for distillation quantity) with the dissolved and suspended catalyst in it is returned to-the ethylene chlorination reactor (West Germany Application 3247988,cl.C 07 C 19/045, C 07 C 17/02,1984).

### DISCLOSURE OF THE INVENTION

The technical result of the invention is the increase of the initial reagents conversion level to the final product and the decrease of by-products due to selectivity increasment of ethylene chlorination to ethylene dichloride.

This result is obtained by using in the process of ethylene dichloride production the catalyst of the general formula MClₙLm, where Cl - chlorine,M- iron (n=2;3),aluminium (n=3),tin (n=2;4), antimony (n=5) and L- trimethylamine (m=1;3),triethylamine (m=1;3), pyridine (m=1;2;3;4),1-(2-ethyl chloride)trimethylammonium chloride (m=1),1-(2-ethyl chloride)triethylammoniumchloride (m=1),1(2-ethyl chloride)pyridinium chloride (m=1),pyridinechlorohydrate (m=1). Ethylene dichloride is produced by interaction of chlorine with ethylene in liquid dichloroethane medium at a temperature from 20°C to reaction mass boiling temperature in the presence of the abovementioned catalyst.The content of catalyst in the reaction mass constitutes 0,01-10,0% mass.

Chlorination of ethylene is carried out in the reactor at the temperature of 20-75°C in liquid dichloroethane medium in the presence of catalyst of MClṅLm formula,the obtained product with the catalyst is fed to the reactor, operating at the reaction mass boiling temperature of 84-90°C,from the top of the reactor ethylene dichloride is extracted in the vapour state.The evaporated dichloroethane is condensed, then part of it required for maintaining the assigned level andtemperature of the reation mass is returned to the chlorination reactor at the reaction mass boiling temperature. The remaining part of dichloroethane is taken as a final product.

The catalyst accumulating in the chlorination reactor at the boiling temperature in the solution of ethylene dichloride in the quantity of 2-12 wt.% based on the quantity of dichloroethane fed to the reactor, is directed to the chlorination reactor at the temperature of 20-75°C.

The ratio of ethylene dichloride quantity, fed for evaporation from the ethylene chlorination reactor at the temperature of 20-75°C to the quantity of the same produced directly in the chlorination reactor at the temperature of reaction mass boiling can be varied in the range from 1 to 4,5.

Chlorination of ethylene to ethylene dichloride have been carried out in the laboratory and industrial facilities.

Catalysts are prepared by one of the two methods (A and B).

According to Method A the catalyst is produced in a separate reactor by mixing of calculated quantities of metal chloride and nitrogen containing compounds in ethylene dichloride which is then fed to the ethylene chlorination reactor.

According to Method B catalyst is produced in a separate reactor or directly in the ethylene chlorination reactor by mixing calculated quantities of metal chloride and amine (pyridine or trimethylamine or triethylamine) in ethylene dichloride,then chlorine and ethylene are fed to the resulting mixture.The synthesis of catalyst is carried out at the temperature from 20°C to ethylene dichloride boiling temperature until the initial reagents are spent.

The efficiency of utilized catalysts in producing ethylene dichloride by ethylene chlorination was proved by the following aspects:content ( wt.%) of final product and by-side chlorination products in raw dichloroethane removed from the reactor without preliminary purification; catalyst service life; catalyst consumption for the unit (kg) of produced ethylene dichloride.

### DESCRIPTION OF THE PREFERRED EMBODIMENTS

For better understanding of the present invention the particular examples of its utilization are presented below, the ordinal numbers of catalysts are provided in brackets under digits 1-24.

Example 1. 1000g of 0,1% solution of catalyst system consistingof iron trichloride and pyridine in molar ratio 1:1 (1) in ethylene dichloride is charged to the glass reactor of bubbling type 1,51 volume.Chlorine and ethylene are fed at the flow rate of 10,0 and 10,51/hour, respectively.The temperature in the reaction zone is maintained at the level of 35±2°C.85-90% of ethylene dichloride is distilled off from raw ethylene dichloride.The remainder of ethylene dichloride with the dissolved catalyst system is recycled to the reactor.Composition and output of obtained raw ethylene dichloride are provided in Table 1.

Examples 2-24.Chlorination of ethylene is performed as in Example 1,only the following catalyst systems are used:iron trichloride pyridin (mol.ratio 1:4)(2),iron dichloride-pyridin (mol.ratio 1:1)(3),aluminium trichloride-pyridine (mol.ratio 1:1)(4),aluminium trichloride-pyridine (mol.ratio 1:3)(5),tin dichloride-pyridine (mol ratio 1:2)(6),tin tetrachloride-pyridine (mol.ratio 1:1)(7),antimony pentachloride-pyridine (mol.ratio 1:1)(8),iron trichloride-triethylamine (mol.ratio 1:1)(9),iron trichloride-triethylamine (mol.ratio 1:3)(10),aluminium trichloride-triethylamine (mol.ratio 1:1)(11), aluminium trichloride-triethylamine (mol.ratio 1:3)(12),iron trichlo ride-trimethylamine (mol.ratio 1:1)(13),iron trichloride-trimethylamine (mol.ratio 1:3)(14),aluminium trichloride-trimethylamine (mol. ratio 1:1)(15),aluminium trichloride-trimethylamine (mol.ratio 1:3) (16),iron trichloride-1-2(ethylchloride)pyridinium chloride (mol. ratio 1:1)(17),aluminium trichloride-1-(2-ethyl chloride)-pyridinium chloride (mol.ratio 1:1)(18),iron trichloride-1-(2-ethyl chloride) triethylammonium chloride (mol.ratio 1:1)(19),aluminium trichloride-1-(2-ethylchloride)triethylammonium chloride (mol.ratio 1:1) (20), iron trichloride-1-(2-ethylchloride)trimethylammonium chloride (mol. ratio 1:1(21),aluminium trichloride-1-(2-ethylchloride)-trimethylammonium chloride (mol.ratio 1:1)(22),iron trichloride-pyridine chlorohydrate (mol.ratio 1:1)(23),aluminium trichloride-pyridine chlorohydrate (mol.ratio 1:1) (24).

Composition and output of raw ethylene dichloride are shown in Table 1.

Example 25. 2000g of 1,0% catalyst system (1) solution in ethylene dichloride is charged into the glass reactor of bubbling type (H=1000mm,D=70mm).Chlorine and ethylene are fed at the flowrate of 15,0 and 15,51/h,respectively.In the reaction zone the reaction mixture boiling temperature is maintained at the level of 84-86°C. The produced raw ethylene dichloride is extracted in the vapour state. Composition and output of the produced raw ethylene dichloride are provided in Table 2.

Examples 26-47 Ethylene chlorination is performed as in Example 25,only the catalyst systems 2-5 and 7-24 are used.Composition and output of raw ethylene dichloride are shown in Table 2.

**Table 1**

| Results of ethylene chlorination at a temperature of 33-37oC | | | | | |
|---|---|---|---|---|---|
| Examples Nos. | Catalyst system | Final product composition, wt.% | | | Ethylene dichloride yield,% |
| | | Ethylene dichloride | Ethylene trichloride | Low boiling impurities* | |
| 1 | 2 | 3 | 4 | 5 | 6 |
| 1 | 1 | 99,95 | 0,01 | 0,04 | 99,9 |
| 2 | 2 | 99,95 | 0,01 | 0,04 | 99,9 |
| 3 | 3 | 99,87 | 0,07 | 0,05 | 99,7 |
| 4 | 4 | 99,94 | 0,02 | 0,04 | 99,9 |
| 5 | 5 | 99,94 | 0,02 | 0,04 | 99,9 |
| 6 | 6 | 99,83 | 0,12 | 0,05 | 99,6 |
| 7 | 7 | 99,89 | 0,06 | 0,05 | 99,8 |
| 8 | 8 | 99,94 | 0,02 | 0,04 | 99,8 |
| 9 | 9 | 99,90 | 0,05 | 0,05 | 99,9 |
| 10 | 10 | 99,93 | 0,03 | 0,04 | 99,9 |
| 11 | 11 | 99,90 | 0,04 | 0,06 | 99,9 |
| 12 | 12 | 99,91 | 0,04 | 0,05 | 99,9 |
| 13 | 13 | 99,91 | 0,04 | 0,05 | 99,9 |
| 14 | 14 | 99,90 | 0,04 | 0,06 | 99,9 |
| 15 | 15 | 99,92 | 0,03 | 0,05 | 99,9 |
| 16 | 16 | 99,93 | 0,02 | 0,05 | 99,9 |
| 17 | 17 | 99,94 | 0,02 | 0,04 | 99,9 |
| 18 | 18 | 99,93 | 0,03 | 0,05 | 99,9 |
| 19 | 19 | 99,92 | 0,03 | 0,05 | 99,9 |
| 20 | 20 | 99,91 | 0,04 | 0,05 | 99,9 |
| 21 | 21 | 99,92 | 0,03 | 0,05 | 99,9 |
| 22 | 22 | 99,90 | 0,04 | 0,06 | 99,9 |
| 23 | 23 | 99,93 | 0,03 | 0,04 | 99,9 |
| 24 | 24 | 99,91 | 0,04 | 0,05 | 99,9 |

| | | | | | |
|---|---|---|---|---|---|
| * - Methyl chloride,chloroform | | | | | |

**Table 2**

| Ethylene chlorination results at reaction mass boiling temperature of 84-85°C | | | | | |
|---|---|---|---|---|---|
| Examples Nos. | Catalysts system | Final product composition,wt.% | | | Ethylene dichloride yield,% |
| | | Ethylene dichloride | Ethylene trichloride | Low boiling impurities* | |
| 1 | 2 | 3 | 4 | 5 | 6 |
| 25 | 1 | 99,92 | 0,03 | 0,04 | 99,9 |
| 26 | 2 | 99,91 | 0,04 | 0,05 | 99,9 |
| 27 | 3 | 99,85 | 0,10 | 0,05 | 99,6 |
| 28 | 4 | 99,90 | 0,06 | 0,04 | 99,9 |
| 29 | 5 | 99,92 | 0,03 | 0,05 | 99,9 |
| 30 | 7 | 99,86 | 0,09 | 0,05 | 99,7 |
| 31 | 8 | 99,91 | 0,05 | 0,04 | 99,9 |
| 32 | 9 | 99,87 | 0,07 | 0,06 | 99,8 |
| 33 | 10 | 99,89 | 0,06 | 0,05 | 99,9 |
| 34 | 11 | 99,87 | 0,08 | 0,05 | 99,8 |
| 35 | 12 | 99,88 | 0,07 | 0,05 | 99,8 |
| 36 | 13 | 99,89 | 0,06 | 0,05 | 99,9 |
| 37 | 14 | 99,88 | 0,07 | 0,05 | 99,8 |
| 38 | 15 | 99,86 | 0,09 | 0,05 | 99,7 |
| 39 | 16 | 99,86 | 0,09 | 0,05 | 99,7 |
| 40 | 17 | 99,86 | 0,08 | 0,06 | 99,7 |
| 41 | 18 | 99,93 | 0,03 | 0,04 | 99,9 |
| 42 | 19 | 99,91 | 0,04 | 0,05 | 99,9 |
| 43 | 20 | 99,88 | 0,07 | 0,05 | 99,8 |
| 44 | 21 | 99,87 | 0,07 | 0,06 | 99,8 |
| 45 | 22 | 99,88 | 0,07 | 0,05 | 99,8 |
| 46 | 23 | 99,86 | 0,08 | 0,06 | 99,7 |
| 47 | 24 | 99,91 | 0,04 | 0,05 | 99,9 |

| | | | | | |
|---|---|---|---|---|---|
| * Chloromethyl,chloroform | | | | | |

Example 48.9,50m³ (12000kg) of ethylene dichloride and 120kg of catalyst system 17 are charged into the industrial reactor of column type (H=8400mm,D=1400mm). 1000m³/hour of 95-97% chlorine (based on 100% chlorine content) and 1050-1100m³/hour of ethylene are fed to the reactor.The reaction mixture due to the reaction heat is heated up to the boiling temperature of 85-90°C Ethylene dichloride,evaporating from the reactor, is condensed, then partially is recycled to the reactor for maintaining the reaction mass set level and temperature.The other part,equal in quantity of synthesized ethylene dichloride (4419kg/hour),is withdrawn as a product. 20-30kg of the same catalyst system in the form of 10% solution in ethylene dichloride is added to the reaction mass every 250-500 hours for maintaining high selectivity.

Catalyst consumption per one ton of the product is 0,023-0,027 kg.

Output per one ton of the product amounts to:
- Ethylene dichloride: 996,0-998,0kg
- 1,1,2-trichloroethane: 0,8-2,6kg
- Low-boiling impurities: 0,4-1,0kg
- Polychlorides and oligomers: 0,035-0,050kg

Example 49. 7,64m³ (9500kg) of ethylene dichloride and 10kg of catalyst system 17 in the form of 10% solution in ethylene dichloride are charged into the industrial reactor of column type with the capacity of 10m³.Chlorine and ethylene are fed to the reactor at the flow rate of 200 and 220m³/hour,respectively (on 100% reagent concentration basis).The temperature in the reactor is kept at 45±3°C. Ready-made ethylene dichloride is withdrawn through overflow in the amount of 884kg per hour.Catalyst system is added to the reactor as 10% solution in ethylene dichloride in the amount of 0,826kg /hour.

For purification from dissolved catalyst system,chlorine and hydrogen chlorine raw ethylene dichloride undergoes water-alkaline flushing and azeotropic drying,at this the catalyst system decomposes and is removed with washing water.

Catalyst consumption per one ton of the product is 0,90-1,10kg.

Output per one ton of the product amounts to:
- Ethylene dichloride: 999,0-999,5kg
- 1,1,2-trichloroethane: 0,2-0,4kg
- Low-boiling impurities: 0,3-0,5kg
- Polychlorides and oligomers: 0,012-0,020kg

Example 50. Chlorination of ethylene is carried out simultaneously by two methods:at boiling of the reaction mass (85-90°C) in the reactor-evaporator with extracting of the final product in a vapour state (similar to the Example 48) and in five reactors at a temperature of 45°C (as in the Example 49) with feeding of 4419kg/h raw ethylene dichloride produced here to the reactor-evaporator. 8838kg/h of ethylene dichloride and 170-500kg/h of reaction mass depending on catalyst concentration in it are withdrawn from the reactor-evaporator.The above mentioned quantity of the reaction mass is sent to the reactors operating at a temperature of 45°C for providing 0,05-1,00 wt.% catalyst system concentration.At this the ratio of ethylene dichloride quantity,fed for evaporation from the reactors operating at 45°C ,to ethylene dichloride quantity produced in the reactor-evaporator,is equal to 1.

Catalyst consumption per one ton of the product is 0,016-0,024 kg.

Output per one ton of the product amounts to:
- Ethylene dichloride: 998,0-998,7kg
- 1,1,2-trichloroethane: 0,4-1,2kg
- Low-boiling impurities: 0,4-0,7kg
- Polychlorides and oligomers: 0,024-0,035kg

Some additional characteristics of the process are presented in Table 3.

Example 51. Ethylene chlorination is carried out in the same way as in Example 50 but ethylene dichloride produced in 14 reactors operating at 45°C, is fed for evaporatioₙto the reactor-evaporator at the flow-rate of 1237kg/h. 16781kg/h of ethylene dichloride and 690-1400kg/h of reaction mass depending on catalyst system concentration in it are withdrawn from the reactor-evaporator.The above mentioned quantity of the reaction mass is sent to the reactors operating at a temperature of 45°C for providing 0,05-1,00wt.% catalyst system concentration in the reactors.At this the ratio of ethylene dichloride quantity,fed for evaporation from the reactors operating at 45°C to ethylene dichloride quantity,produced in the reactor-evaporator,is equal to 2,8.

Catalyst consumption per one ton of the product is 0,010-0,012 kg.

Output per one ton of the product amounts to:
- Ethylene dichloride: 998,0-999,0kg
- 1,1,2-trichloroethane: 0,3-0,7kg
- Low-boiling impurities: 0,4-0,7kg
- Polychlorides and oligomers: 0,018-0,028kg

Some additional characteristics of the process are given in Table 3.

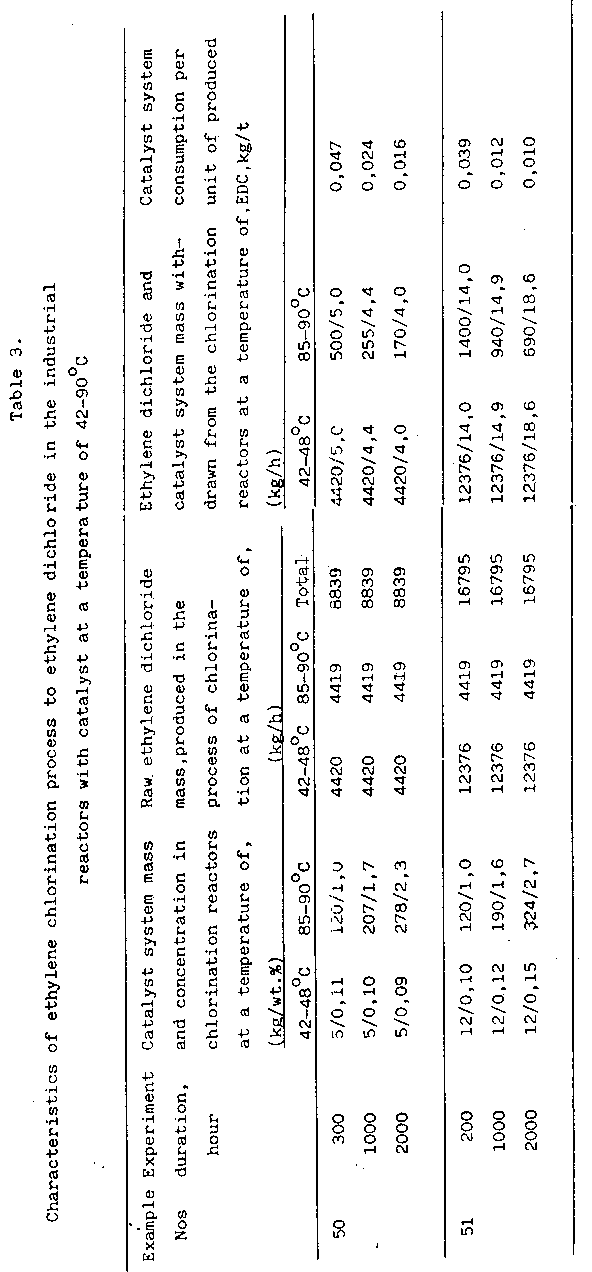

## Claims

1. The process for ethylene dichloride production comprising chlorine and ethylene interaction in liquid ethylene dichloride medium at a temperature from 20°C to the reaction mass boiling temperature in the presence of a catalyst wherein the compounds of general formula MCln -Lm,in which Cl- chlorine;M -iron (n=2;3), aluminium (n=3),tin (n=2;4),antimony (n=5) and L -trimethylamine (m=1;3),triethylamine (m=1;3),pyridine (m=1;2;3;4:),1-(2-chloroethyl)trimethylammonium chloride (m=1),1-(2-chloroethyl)triethylammonium chloride (m=1),1-(2-chloroethyl)pyridinium chloride (m=1), pyridinchlorohydrate (m=1),are used as a catalyst.

2. The process of claim 1 in which chlorination of ethylene is carried out in the reactor at a temperature of 20-75°C,the obtained product with the catalyst is fed to the ethylene chlorination reactor operating at the reaction mass boiling temperature, ethylene dichloride in a vapour state is withdrawn from the top of the reactor as a final product and the catalyst is taken out from the bottom in the solution of ethylene dichloride and recycled to the chlorination reactor at a temperature of 20-75°C.

3. The process of claim 1 wherein the catalyst content in the reaction mass of the process is 0,01-10,0wt.%.

4. The process of claim 1 wherein the process is carried out at the ratio of ethylene dichloride produced at 20-75°C and at reaction mass boiling temperature equal to 1-4,5:1.
